# EUROPEAN PATENT SPECIFICATION

(11) **EP 2 373 319 B1**
(45) Date of publication and mention of the grant of the patent: **31.07.2013**
(21) Application number: 10717870.9
(22) Date of filing: 04.01.2010
(51) Int. Cl.: A61K 31/554, A61K 9/20, A61K 9/28

(54) **SUSTAINED RELEASE PHARMACEUTICAL COMPOSITION OF QUETIAPINE AND PROCESS FOR PREPARATION THEREOF**
PHARMAZEUTISCHE ZUSAMMENSETZUNG AUS QUETIAPIN MIT VERZÖGERTER FREISETZUNG UND VERFAHREN ZU IHRER HERSTELLUNG
COMPOSITION PHARMACEUTIQUE À LIBÉRATION PROLONGÉE À BASE DE QUÉTIAPINE ET SON PROCÉDÉ DE PRÉPARATION

(30) Priority: 05.01.2009 IN MU00232009
(43) Date of publication of application: 12.10.2011
(73) Proprietor: Torrent Pharmaceuticals Limited, 380 009 Ahmedabad (IN)
(72) Inventor: VAYA, Navin, Ishwarlal, Bhat 382 428 (IN); SONI, Narendra, Dulichand, Bhat 382 428 (IN); JOSHI, Baxis, Rameshchandra, Bhat 382 428 (IN)
(74) Representative: Le Coupanec, Pascale A.M.P.
(86) International application number: PCT/IN2010/000002
(87) International publication number: WO 2010/082220

(56) References cited:
- EP-A1- 0 240 228
- WO-A1-97/45124
- WO-A1-03/039516
- WO-A1-2008/090569
- WO-A1-2010/012490
- WO-A2-2007/086079
- US-A1- 2005 158 383
- GUPTA V K; HARIHARAN M; WHEATLEY T A; PRICE J C: "Controlled-release tablets from carrageenans: effect of formulation, storage and dissolution factors" EUROPEAN JOURNAL OF PHARMACEUTICS AND BIOPHARMACEUTICS, ELSEVIER SCIENCE PUBLISHERS B.V., AMSTERDAM, NL, vol. 51, no. 3, 1 May 2001 (2001-05-01), pages 241-248, XP004239485 ISSN: 0939-6411

## Description

### FIELD OF THE INVENTION

The present invention relates to sustained release pharmaceutical composition of quetiapine, and process for preparing such composition. More particularly, it relates to sustained release pharmaceutical composition of quetiapine comprising a non-gelling agent namely λ-currageenan, magnesium oxide and pharmaceutically acceptable excipients.

### BACKGROUND OF THE INVENTION

US Patent No. 4,879,288 disclose 11-[4-[2-(2-hydroxyethoxy) ethyl] -1-piperazinyl] dibenzo [b, f] [1, 4] thiazepine as a novel antipsychotic drug of dibenzothiazepine class suitable for treatment of various psychotic disorders and having much reduced incidence of side effects. It is commonly known as quetiapine and is used for the treatment of schizophrenia and acute manic episodes associated with bipolar I disorder. Quetiapine is thought to exert its efficacy in schizophrenia through a combination of dopamine type 2 (D₂) and serotonin type 2 (5HT₂) antagonism. Quetiapine is marketed as its fumarate salt by AstraZeneca Pharmaceutical LP under the brand-name Seroquel^{®}.

Due to high degree of bioavailability and rapid metabolism, quetiapine typically has to be administered multiple times. Such multiple administrations, however, may result in problems related to patient compliance. Accordingly, it may be advantageous to provide a composition which allows for sustained release of quetiapine. Such sustained release may provide a generally uniform and constant rate of release over an extended period of time which may achieve a stable and desired blood (plasma) level of quetiapine without the need for frequent administration. The art discloses some approaches for preparing sustained release pharmaceutical compositions of quetiapine.

US Patent no. 5,948,437 assigned to Zeneca Ltd. discloses a sustained release composition comprising quetiapine or a pharmaceutically acceptable salt thereof, and a gelling agent together with one or more pharmaceutically acceptable excipients. The gelling agent is described to a hydrophilic substance, which forms a gel when in contact with water. Preferably, the gelling agent is hydroxypropyl methylcellulose. Extended release tablets of quetiapine fumarate sold under the tradename SEROQUEL^{®} XR by AstraZeneca are believed to be based on the teachings of US 5,948,437. How ever in the same patent the applicant described for several reasons that designing a sustained release formulation of water soluble active agents and their pharmaceutically acceptable salts such as quetiapine fumarate, using a gelling agent would be very difficult. Such kind of composition comprising active ingredient which is soluble in water tend to generate a sustained release product which is susceptible to a phenomenon known as dose dumping. That is, release of the active ingredient is delayed for a time but once release begins to occur, the rate of release would de very high. Moreover, fluctuations tend to occur in the plasma concentrations of the active ingredient which increases the likelihood of toxicity.

Hence development of such kind of formulation may often involve strenuous procedures and require several days to optimize, which may not be commercially a viable option.

The WO 2005/41935 patent application assigned to Alpharma Inc. discloses dosage forms of quetiapine and its salts, including wax dosage forms, press-coat dosage forms, and sprinkle dosage forms. It also discloses pulsed release dosage forms of quetiapine and its salts. In one of its preferred embodiment, it discloses a sustained release solid dosage formulation comprising a matrix, wherein the matrix comprises a therapeutically effective amount of quetiapine or a pharmaceutically acceptable salt thereof, and a wax material.

The WO 2007/86079 patent application assigned to Astron Research Ltd. discloses once a day sustained release solid oral dosage form of quetiapine and their pharmaceutically acceptable salts comprising a channelizer, rate controlling polymer and suitable pharmaceutically acceptable excipients. The preferred channelizers are electrolytes and osmotic agents.

The WO 2007/110878 patent application assigned to Panacea Biotec Ltd. discloses a sustained release pharmaceutical composition comprising at least one poorly soluble active agent(s), at least one solubilizer, a release rate controlling polymer system consisting of an acid-soluble polymer and a pH-dependent polymer, and optionally other pharmaceutically acceptable excipients. The preferred solubilizers are hydrophilic surfactants. lipophilic surfactants, or mixtures thereof.

The WO 2007/00778 patent application assigned to Panacea Biotec Ltd. discloses a modified release pharmaceutical composition comprising at least one active agent(s); a polymer system comprising at least two swellable pH independent polymers wherein at least one is hydrophilic; and optionally other pharmaceutically acceptable excipients.

The WO 2007/133583 patent application assigned to Mallinckrodt Inc. discloses a modified release solid dosage form comprising a matrix core and a modified release coating. The matrix core comprises a hydrophobic material and a water soluble pharmaceutical agent such as quetiapine. The modified release coating surrounds the matrix core, and comprises a hydrophobic polymer and a hydrophilic pore-forming agent. The dosage form exhibits a zero-order release profile upon dissolution. The hydrophilic pore-forming agent in modified release coating is described to be essential for zero-order release of drug from the dosage form.

The WO 2006/81347 patent application assigned to Elan Pharma discloses formulations that deliver quetiapine in a pulsed or bimodal manner. The formulations comprise an immediate release and a modified release component. The components are described to be selected from different populations of particles or mini-tablets.

The US 2008/221078 patent application discloses pharmaceutical composition of quetiapine, an intimate mixture of polyvinyl acetate and polyvinyl pyrrolidone (i.e. Kollidon SR^{®}) and an acid.

Thus, a need exists in the art for a simpler design of sustained release formulations of soluble medicaments such as quetiapine using conventional manufacturing procedures. Accordingly the present inventors have evaluated several types of non-gelling agents for the purpose of imparting sustained release activity to quetiapine formulations found that desirable dissolution profiles across different dissolution media was achieved, most desirably by the formulations using A -carrageenan as a release modifying agent, and magnesium oxide

### SUMMARY OF THE INVENTION

The present text describes a sustained release pharmaceutical composition comprising:
a) quetiapine,
b) non-gelling agent
c) at least one pharmaceutically acceptable excipient.

The present text describes a sustained release pharmaceutical composition wherein the core comprising:
a) quetiapine,
b) non-gelling agent
c) at least one pharmaceutically acceptable excipient.

The present text describes a sustained release pharmaceutical composition wherein the core comprising:
a) quetiapine,
b) carrageenan; and
c) at least one pharmaceutically acceptable excipient.

The present text describes a sustained release pharmaceutical composition wherein the core comprising:
a) quetiapine,
b) carrageenan,
c) at least one hydrophobic release-modifying polymer; and
d) at least one pharmaceutically acceptable excipient.

The present text describes a sustained release pharmaceutical composition wherein the core comprising:
a) quetiapine,
b) carrageenan,
c) at least one hydrophobic release-modifying polymer; and
d) at least one basic agent
e) at least one pharmaceutically acceptable excipient.

The present invention discloses a sustained release pharmaceutical composition wherein the core comprising:
a) quetiapine,
b) λ -carrageenan as carrageenan;
d) Magnesium Oxide as at least one basic agent; and
e) at least one pharmaceutically acceptable excipient.;and optionally a coating applied on the core.

Another embodiment of the present invention discloses a sustained release pharmaceutical composition as described above wherein the core is optionally coated with film-forming polymer or a release-modifying polymer.

Yet another embodiment discloses a process for preparing a sustained release pharmaceutical composition, wherein the process comprises:
(i) mixing quetiapine, carrageenan and at least one pharmaceutically acceptable excipient;
(ii) granulating the mixture of step (i) with a solvent;
(iii) drying the granules;
(iv) optionally coating the granules obtained in step (iii) with a hydrophobic release-modifying polymer;
(v) mixing the granules of step (iii) or (iv) with at least one pharmaceutically acceptable excipient; and
(vi) compressing the mixture of step (v) into a tablet.
(vii) and optionally coating the tablets of step (vi)

### DETAILED DESCRIPTION OF THE INVENTION

The term "sustained release" as described herein is intended for a composition which provides the desired therapeutic effect of quetiapine for a period of more than 12 hours, for e.g. for 24 hours.

The term "quetiapine" as described herein includes quetiapine free base, as well as pharmaceutically acceptable salts, prodrugs, metabolites thereof, or enantiomers thereof. It also includes hydrates, solvates and polymorphs of quetiapine free base or pharmaceutically acceptable salts thereof; or mixtures thereof. The preferred salt is quetiapine fumarate. Quetiapine may be present in an amount ranging from 1 % to 70 % by weight of the composition.

Carrageenan is a naturally occurring family of polysaccharides extracted from certain types of red seaweed belonging to the Rhodophyceae family. It is a high molecular weight polysaccharide made up of repeating galactose and 3, 6 anhydrogalactose units, both sulfated and nonsulfated. The units are joined by alternating α1-3 and β1-4 glycosidic linkages. Carrageenan is basically of three types: Iota, Kappa and Lambda.

Carrageenan is generally used in the oral solid dosage forms such as in tablet formulation to impart release characteristics. Carrageenan being hydrophilic absorbs water from surroundings when placed in aqueous liquids and there by forming a viscous gel, this gel in turn slows down the release of active ingredient embedded in it and provides a sustained release of drug from the formulation.

Unlike all the grades of Carrageenan, A -Carrageenan (Example: marketed under the trade name Viscarin GP^{®}) is a hydrophilic agent which shows no gelling properties upon absorption of water from surroundings when placed in aqueous liquids. Such kind of grade of carrageenan can still impart sustained release properties to a solid oral dosage form such as tablet.

Accordingly, an aspect of the present invention provides sustained release solid oral dosage forms of quetiapine, wherein A -Carrageenan is present in an amount that imparts sustained release properties to the dosage form.

Apart from A -carrageenan, there are other non gelling release modifying agents known in the art for their sustained release properties which include, but not limited to, povidone, poly ethylene glycol, co-povidone, polyvinyl acetate, polyvinyl acetate-based copolymers, and any hydrophobic modified cellulose derivatives, non-gelling polysaccharides, non-gelling modified polysaccharides, cellulose acetate phthatate, cellulose acetate succinate, hypromellose phthalate, hypromellose acetate succinate, polyvinylacetate phthalate, hydroxyethyl cellulose phthalate, cellulose acetate maleate, cellulose acetate trimellitate, cellulose acetate butyrate, cellulose acetate propionate, polymethacrylates such as methacrylic acid-methyl methacylate co-polymers methacrylic acid-ethylacrylate co-polymers, methacrylic acid-methyl acrylate-methyl methacrylate co-polymers, and the like or combinations comprising at least one of the foregoing.

Accordingly, in an aspect of the present invention provides sustained release solid oral dosage forms of quetiapine, wherein the dosage form comprises quetiapine and at least one non-gel forming agent in an amount that imparts sustained release properties to the dosage form.

The "hydrophobic release-modifying polymer" as described herein refers to any hydrophobic polymer which is capable of providing sustained release of quetiapine. The hydrophobic release-modifying polymer may be a pH-dependent or a pH-independent polymer or mixtures thereof. The hydrophobic release-modifying polymer include, but not limited to, polymethacrylates; phthalates like cellulose acetate phthalate, hydroxypropyl methylcellulose phthalate, hydroxypropyl cellulose phthalate, polyvinyl acetate phthalate; hydroxypropyl methylcellulose acetate succinate, cellulose acetate, cellulose acetate butyrate, cellulose triacetate, polyethylene, polyvinylchloride, polyethylene vinyl acetate, polydimethyl siloxane, polyether urethane, stearyl alcohol, and the like. Preferably, the hydrophobic release-modifying polymer include, but not limited to, various pharmaceutically acceptable polymethacrylates sold under the brand name EUDRAGIT®**.** The hydrophobic release-modifying polymer and carrageenan may be added intragranularly or extragranularly or both.

The above mentioned non-gelling agents of the present invention can be present either in the core or in the coating or in both.

Accordingly in one aspect of the present invention the non-gelling agents and hydrophobic agents are present in the core that imparts sustained release properties to quetiapine compositions.

In yet another aspect of the present invention the non-gelling agents and hydrophobic agents provide a sustained release coating to quetiapine compositions.

The compositions of the present invention may comprise A -Carrageenan from 1% to about 70% weight of the composition, more preferably 1% to about 60%, or from 1% to about 50% and most preferably from 1% to about 40% of the total weight of the composition.

The pharmaceutical compositions as described herein may comprise one or more pharmaceutically acceptable excipients selected from diluent, disintegrant, binder, buffering agent, glidant, lubricant, plasticizer, opacifying agent and anti-tacking agent.

As used herein, the term "diluents" is intended to mean inert substances used as fillers to create the desired bulk, flow properties, and compression characteristics in the preparation of dosage form. Diluent include, but not limited to, powdered cellulose, microcrystalline cellulose, diabasic calcium phosphate, pregelatinized starch, magnesium oxide, ammonium alginate, calcium phosphate tribasic, fumaric acid, glyceryl palmitostearate, hydrogenated vegitable type I, isomalt, kaolin, lactitol, cellulose acetate, ethylcellulose, erythritol, sugars such as lactose, sucrose, dextrose, dextrin, fructose, xylitol, and the like; sugar alcohols such as mannitol, maltose, polydextrose, maltodextrin, sorbitol or erythritol; calcium phosphate, calcium carbonate, calcium sulphate; magnesium carbonate, medium chain triglycerides, polymethacrylates, simethicone, sodium alginates, sodium chlorides, sulfobutylether beta-cyclodextrin, talc, tragacanth, trehalose and mixtures thereof. The diluent may be present in an amount ranging from 1 % to 90 % by weight of the composition.

As used herein, the term "binder" is intented to mean inert substance used to form the bridge between the drug particles with other excipients. Binder may includes, but not limited to, acacia, agar, alginic acid, carbomers, carboxymethylcellulose sodium, carrageenan, cellulose acetate phthalate, ceratonia, chitosan, confectioner's sugar, copovidone, cottonseed oil, dextrates, dextrin, glyceryl behenate, gelatin, guar gum, hydrogenated vegitable oil type I, hydroxyethyl cellulose, hydroxyethylmethyl cellulose, hydroxypropyl cellulose, hydroxypropryl starch, hypromellose, inulin, lactose, liquid glucose, magnesium aluminium silicate, maltodextrin, maltose, methylcellulose, poloxamer, polycarbophil, polydextrose, polyethylene oxide, polymethycrylates, povidone, sodium alginate, starch, stearic acid, sucrose, sunflower oil, zein.

"Disintegrant" herein used to provides a solid oral dosage form such as tablet of the present invention to facilitate drug release. Examples of such disintegrants are but not limited to, calcium alginate, carboxymethylcellulose calcium, carboxymethylcellulose sodium, cross-linked carboxymethylcellulose sodium, cross-linked polyvinyl pyrrolidone, cellulose, chitosan, colloidal silicon dioxide, docusate sodium, guar gum, magnesium aluminium silicate, methylcellulose, polacrilin potassium, starch, sodium starch glycolate, low-substituted hydroxypropyl cellulose, pregelatinized starch, sodium alginate; or mixtures thereof. The disintegrant may be present in an amount ranging from 1 % to 15 % by weight of the composition.

Buffering agents and basifying agents used in the present invention to provide better stability to the compositions under normal and stressed conditions examples of such buffering and basifying agents include, but not limited to, organic acids and its salts, mineral acids, alkali metal phosphates, carbonates, hydroxides, base and the like; and mixtures thereof Preferably, buffering agent is selected from ammonia solution, calcium carbonate, calcium phosphate, benzoic acid, citric acid, tartaric acid, succinic acid, sodium carbonate, sodium citrate, sodium or potassium hydroxide, dibasic sodium phosphate, diethanolamine, malic acid, monobasic sodium phosphate, monoethanolamine, monosodium glutamate, phosphoric acid, potassium citrate, sodium citrate, sodium bicarbonate, sodium acetate, sodium borate, sodium citrate dehydrate, sodium hydroxide, sodium lactate, triethanolamine, disodium hydrogen ortho phosphate, and the like; or mixtures thereof. Preferably, the buffering agent is sodium citrate. The buffering agent may be present in an amount ranging from 0.1 % to 15 % by weight of the composition. Suitable basic agent are pharmaceutically acceptable compound such as but not limited to, alkaline earth metal carbonate, alkali metal carbonates, alkaline earth metal bicarbonates, alkali metal bicarbonates, alkali metal hydroxide, alkali earth metal hydroxide, alkali metal oxide & alkali earth metal oxide, which includes, but not limited, magnesium oxide, calcium carbonate, sodium carbonate, potassium carbonate, sodium bicarbonates, potassium bicarbonates, ammonia solution, diethanolamine, monoethanolamine, potassium citrate, sodium borate, sodium citrate dehydrate, triethanolamine, sodium hydroxide & potassium hydroxide or mixtures thereof. Preferably, the basifying agent is magnesium oxide. The basifying agent may be present in an amount ranging from 0.1 % to about 50 % by weight of the composition, more preferably 1 % to about 40%, or 1% to about 30%.

A lubricant may be added to the pharmaceutical compositions of the present invention to reduce adhesion and/or ease the release of the product from example the die, punch. Lubricant or glidant include, but not limited to, talc, metallic stearates such as magnesium stearate, calcium stearate, zinc stearate; colloidal silicon dioxide, finely divided silicon dioxide, stearic acid, canola oil, hydroxyethyl cellulose, lauric acid, leucine, mineral oil, poloxamers, polyvinyl alcohol, hydrogenated cator oil, light mineral oil, magnesium sulfate, medium chain triglycerides, myristic acid, polyethylene glycol, potassium benzoate, sodium benzoate, sodium lauryl sulfate, hydrogenated vegetable oil, glyceryl palmitostearate, glyceryl monostearate, sodium stearyl fumarate, glyceryl behenate, sodium stearyl fumarate, magnesium trisilicate; or mixtures thereof.

The lubricant or glidant may be present in an amount ranging from 0.1 % to 10 % by weight of the composition.

As used herein the term "coating" refers a polymer coating which is capable of forming a film on to the core or a layer formed to impart modified release characteristics to the core The coating material may be present in an amount ranging from 0.1 % to 20 % by weight of the composition.

As used herein, the term, "plasticizer" should be able to provide the desired plasticity to the coating. Plasticizer as described herein include, but not limited to, propylene glycol, polyethylene glycol, triethyl citrate, acetyl triethyl citrate, diethyl phthalate, dibutyl phthalate, acetyltributyl citrate, benzyl benzoate, cellulose actate phthalate compatible, chlorbutanol, dextrin, diethyl phthalate, dimethyl phthalate, glycerin, glycerin monostearate, hypromellose phthalate, mannitol, mineral oil and lanolin alcohol, palmitic acid, 2-pyrrolidine, sorbitol, stearic acid, triacetin, tributyl citrate, triethanolamine, dibutyl sebacate; or mixtures thereof.

Plasticizer is generally used in the coating to increase the flexibility and strength of the coat. The plasticizer may be present in an amount ranging from 0.1 % to 20 % by weight of the composition.

Anti-tacking agents may improve the flow characteristics of the compositions and thereby reduce the problem of film adhering to a user's mouth or to other units of film. Anti-tacking agent as described herein include, but not limited, talc, finely divided silicon dioxide, glyceryl monostearate, and the like. The anti-tacking agent may be used in the coating to aid bulk build-up and form a smooth surface. The anti-tacking agent may be present in an amount ranging from 0.1 % to 20 % by weight of the composition.

An opacifier is a substance added generally in tablet coating composition in order to make the system opaque, Opacifying agent as described herein include, but not limited, titanium dioxide, iron oxides, aluminum stearate, calcium carbonate, zinc stearate, ethylene glycol palmitostearate, and the like. The opacifying agent may be present in an amount ranging from 0.1 % to 10 % by weight of the composition.

The pharmaceutical compositions as described herein may be in the form of tablet, capsule, sachet, and the like. The compositions may be prepared by techniques such as wet granulation, dry granulation, drug layering, and the like. For example, quetiapine may be mixed with carrageenan and at least one pharmaceutically acceptable excipient, and the mixture may be granulated with water or an organic solvent in an apparatus, such as rapid mixer granulator or a fluid bed processor to form granules. The organic solvent include, but not limited to, acetone, methanol, Dichloromethane, isopropyl alcohol, and the like; or mixtures thereof. The granules may optionally contain a hydrophobic release-modifying polymer. Alternatively, the mixture of quetiapine, carrageenan and at least one pharmaceutically acceptable excipient may be compacted in a roller compacter and the compacts may be milled to obtain granules. The granules may be coated with a solution or dispersion of the film forming or release-modifying polymer and pharmaceutically acceptable excipients such as binder, plasticizer, anti-tacking agent and opacifying agent. Alternatively, the granules devoid of any coating may be compressed into a tablet which may further be coated with a solution or dispersion of the hydrophobic release-modifying polymer or a film-forming polymer and pharmaceutically acceptable excipients such as binder, plasticizer, anti-tacking agent and opacifying agent. Non limiting examples of coating materials include hydrophilic and hydrophobic excipient such as hydroxypropyl methyl celluloses (hypromellose or HPMC), hydroxypropylcelluloses, ethyl cellulose and other cellulose derivatives, polysaccharide such as all grades of carrageenan, polyvinyl acetates, polyvinyl alcohols, sugars, amino acids, zein, polyvinylpyrrolidones, guar gum and the like; or mixtures thereof. Alternatively, the uncoated or coated granules may be filled in a capsule or a sachet.

In one embodiment, a sustained release tablet is prepared by:
mixing quetiapine, a non-gelling agent and at least one pharmaceutically acceptable excipient selected from diluent, disintegrant, buffering agent, or mixtures thereof;
granulating the mixture with a solvent;
drying the granules;
mixing the granules with a lubricant or glidant;
compressing the mixture into a tablet; and
optionally coating the tablet with a hydrophobic release-modifying polymer or a film-forming polymer.

In another embodiment, a sustained release tablet is prepared by:
mixing quetiapine, carrageenan and at least one pharmaceutically acceptable excipient selected from diluent, disintegrant, buffering agent, or mixtures thereof;
granulating the mixture with a solvent;
drying the granules;
mixing the granules with a lubricant or glidant;
compressing the mixture into a tablet; and
optionally coating the tablet with a hydrophobic release-modifying polymer or a film-forming polymer.

In another embodiment, a sustained release tablet is prepared by:
mixing quetiapine, carrageenan, a hydrophobic release-modifying polymer and at least one pharmaceutically acceptable excipient selected from diluent, disintegrant, buffering agent, or
mixtures thereof;
granulating the mixture with a solvent;
drying the granules;
mixing the granules with a lubricant or glidant; and
compressing the mixture into a tablet.

In further embodiment, a sustained release tablet is prepared by:
mixing quetiapine, carrageenan and at least one pharmaceutically acceptable excipient selected from diluent, disintegrant, buffering agent, or mixtures thereof;
granulating the mixture with a solvent;
drying the granules;
coating the granules with the hydrophobic release-modifying polymer;
mixing the coated granules with a lubricant or glidant; and
compressing the mixture into a tablet.

In further embodiment, a sustained release tablet is prepared by:
mixing quetiapine, carrageenan or non gelling agent and at least one pharmaceutically acceptable excipient;
optimally dry- granulating the mixture of step
optionally coating the granules obtained ;
mixing the mixture as obtained from either of the above steps with at least one pharmaceutically acceptable excipient; and
compressing the mixture into a tablet.
optionally coating the tablet.

The pharmaceutical compositions as described herein may be illustrated by the following examples which are not to be construed as limiting the scope of the invention:

### EXAMPLE 1 (reference)

| **Ingredients** | **Quantity (mg/tab)** |
|---|---|
| Quetiapine fumarate | 230.26 |
| Lactose monohydrate | 38.62 |
| Microcrystalline cellulose (Avicel PH 101*) | 38.62 |
| Sodium citrate | 62.50 |
| PVP K 30 | 20.00 |
| λ -Carrageenan (Viscarin GP 209$) | 100.00 |
| Dichloromethane‡ | q.s. |
| Magnesium stearate | 10.00 |

| | |
|---|---|
| * Avicel PH 101 is marketed by FMC. $ Viscarin GP 209 is marketed by FMC ‡ Evaporates during manufacturing. | |

### Manufacturing process:

1) Quetiapine fumarate, lactose, microcrystalline cellulose sodium citrate and λ - Carrageenan were mixed
2) The blend of step 1 granulated with a binder solution containing polyvinylpyrrolidone (PVP-K-30) in dichloromethane.
3) The granules of step 3 were dried, lubricated with magnesium stearate and compressed into a tablet.

**Table 1: Dissolution profile* of Example 1 in USP Type I Apparatus (Basket) at 200 rpm, 37.0 ± 0.5 °C**

| **Time (hr)** | **% of drug dissolved** |
|---|---|
| 1 | 6.2 |
| 2 | 13 |
| 4 | 24.6 |
| 5 | 28.8 |
| 6 | 32.5 |
| 12 | 51.7 |
| 20 | 67.5 |

| | |
|---|---|
| [*Dissolution Medium: 0- 5 hours: 900 ml of medium containing 0.05 M citric acid + 0.09 M sodium hydroxide to provide pH 4.7-4.8; 5-24 hours: addition of 100 ml of medium containing 0.05 M disodium phosphate + 0.46 M sodium hydroxide to provide the pH 6.8] | |

### EXAMPLE 2

| **Composition** | **mg/tab** |
|---|---|
| **Core** | |
| Quetiapine Fumarate | 230.26 |
| Lactose Monohydrate | 76.87 |
| Microcrystalline Cellulose (Avicel PH 101) | 76.87 |
| Magnesium oxide (Light) | 30.00 |
| PVP K 30 | 20.00 |
| A -Carrageenan (Viscarin GP 209) | 56.00 |
| Dichloromethane ‡ | 150.00 |
| Crospovidone | 10.00 |
| Microcrystalline Cellulose (Avicel PH 102**) | 50.00 |
| Magnesium stearate | 10.00 |

| **Coating** | |
|---|---|
| HPMC 6 cps | 10.08 |
| PEG 400 | 2.02 |
| Titanium dioxide | 4.70 |
| P.Water‡ | 235.00 |
| Total | 576.80 |

| | |
|---|---|
| ** Avicel PH 102 is marketed by FMC. ‡ Evaporates during manufacturing. | |

### Manufacturing Process

1. Quetiapine fumarate, lactose monohydrate, microcrystalline cellulose (Avicel PH 101), magnesium oxide (Light) and A -carrageenan (Viscarin GP 209) were sifted from ASTM 40# sieve and mixed in RMG.
2. The blend of step 1 was granulated using solution of PVP K30 in Dichloromethane.
3. The granules of step 2 were dried in Fluidized Bed Dryer and sized using ASTM 20# sieve.
4. Granules of step 3 were mixed with crospovidone and microcrystalline cellulose (Avicel PH 1020029 in conta blender for 10 minutes.
5. Blend of step 4 was lubricated with magnesium stearate was for 5 minutes.
6. Lubricated blend of step 5 was compressed into tablets.
7. Tablets of step 6 were coated with aqueous dispersion of HPMC, PEG 400 and titanium dioxide.

**Table 2: Dissolution profile* of Example 5 in USP Type I Apparatus (Basket) at 200 rpm, 37.0 ± 0.5 °C**

| **Time (hr)** | **% of drug dissolved** |
|---|---|
| 1 | 7.2 |
| 2 | 16.0 |
| 4 | 38.4 |
| 5 | 47.2 |
| 6 | 58.7 |
| 8 | 83.1 |
| 10 | 92.9 |
| 12 | 94.1 |
| 16 | 94.3 |
| 20 | 94.5 |

| | |
|---|---|
| [*Dissolution Medium: 0- 5 hours: 900 ml of medium containing 0.05 M sodium citrate + 0.09 M sodium hydroxide to provide pH 4.7-4.9; 5-20 hours: addition of 100 ml of medium containing 0.05 M sodium phosphate + 0.46 N sodium hydroxide to provide the pH 6.4-6.8] | |

### EXAMPLE 3

| **Composition** | **mg/tab** |
|---|---|
| **Core** | |
| Quetiapine Fumarate | 230.26 |
| Lactose Monohydrate | 29.87 |
| Microcrystalline Cellulose (Avicel PH 101) | 29.87 |
| Magnesium oxide (Light) | 90.00 |
| PVP K 30 | 20.00 |
| λ -Carrageenan (Viscarin GP 209) | 150.00 |
| Dichloromethane ‡ | 172.00 |
| PVP K 30 | 40.00 |
| Magnesium stearate | 10.00 |

| **Coating** | |
|---|---|
| A -Carrageenan (Viscarin GP 209) | 16.17 |
| PEG 400 | 5.38 |
| Titanium dioxide | 7.70 |
| Iron oxide Yellow | 0.75 |
| P. Water‡ | 226.00 |
| lsopropyl alcohol‡ | 173.90 |
| Total | 630.00 |

| | |
|---|---|
| ‡ Evaporates during manufacturing. | |

### Manufacturing Process

1. Quetiapine fumarate, lactose monohydrate, microcrystalline cellulose (Avicel PH 101) _ and λ -carrageenan (Viscarin GP 209) and magnesium oxide (light) were sifted through 40# sieve and mixed in a RMG.
2. The blend of step 1 was granulated using binder solution i.e. PVP K 30 (Intragranular) in dichloromethane.
3. Granules of step 2 were dried using fluid bed dryer.
4. Dried granules of step 3 were sized through 1.2 mm screen of Oscillating granulator.
5. Sized granules of step 4 were mixed with PVP K 30 (extra granular) using blender for 10 minutes.
6. Magnesium stearate was mixed with blend of step 5 using blender for 5 minutes.
7. Tablets were compressed from lubricated blend of step 6.
8. λ -carrageenan (Viscarin GP 209) was dispersed in isopropyl alcohol followed by polyethylene Glycol 400.
9. Titanium dioxide and ferric oxide yellow was dispersed in purified water and passed through colloid mill for 10 minutes.
10. Contents of step 8 to step 9 were mixed.
11. Compressed tablets were coated using λ-carrageenan coating dispersion of step 10.

**Table 3: Dissolution profile of Example 4 in USP Type II Apparatus (Paddle) at 100 rpm, 37.0 ± 0.5 °C using 1000ml phosphate buffer pH 6.5.**

| **Time (hr)** | **% of drug dissolved** |
|---|---|
| 1 | 2.9 |
| 2 | 11.9 |
| 4 | 29.8 |
| 6 | 43.7 |
| 8 | 56.7 |
| 10 | 69.8 |
| 12 | 79.1 |
| 16 | 90.1 |
| 20 | 92.9 |

### EXAMPLE 4

| **Composition** | **mg/tab** |
|---|---|
| **Core** | |
| Quetiapine Fumarate | 230.260 |
| Lactose Monohydrate | 29.870 |
| Microcrystalline Cellulose (Avicel PH 101) | 29.870 |
| Magnesium oxide light (Compacted) | 90.000 |
| PVP K 30 | 20.000 |
| λ -Carrageenan (Viscarin GP 209) | 150.000 |
| Dichloromethane ‡ | 131.400 |
| PVP K 30 | 40.000 |
| Magnesium stearate | 10.000 |

| **Coating** | |
|---|---|
| λ -Carrageenan (Viscarin GP 209) | 16.170 |
| PEG 400 | 5.380 |
| Titanium dioxide | 7.700 |
| Iron oxide Yellow | 0.750 |
| P. Water‡ | 226.000 |
| Iso propyl alcohol‡ | 173.900 |
| **Total** | **630.000** |

| | |
|---|---|
| ‡ Evaporates during manufacturing. | |

### Manufacturing Process

1. Quetiapine fumarate, lactose monohydrate, microcrystalline cellulose (Avicel PH 101) magnesium oxide light (compacted) and λ -carrageenan (Viscarin GP 209) were sifted through 40# sieve and mixed using Rapid mixer granulator.
2. Blend of step 1 was granulated using binder solution i.e. PVP K 30 (Intragranular) in dichloromethane.
3. Granules of step 2 were dried using fluid bed dryer.
4. Dried granules of step 3 were sized through 0.5 mm screen of Oscillating granulator.
5. Sized granules of step 4 were mixed with PVP K 30 (extra granular) using blender for 10. minutes.
6. Blend of step 5 was lubricated magnesium stearate using blender for 5 minutes.
7. The lubricated blend of step 6 was compressed to Tablets.
8. λ -carrageenan (Viscarin GP 209) was dispersed in lsopropyl alcohol followed by Polyethylene Glycol 400.
9. Titanium Dioxide and Ferric oxide yellow were dispersed in purified water and passed through colloid mill for 10 minutes.
10. Contents of step 8 to step 9 were mixed.
11. Compressed tablets were coated using λ -carrageenan coating dispersion of step 10.

**Table 4: Dissolution profile of Example 4 in USP Type II Apparatus (Paddle) at 100 rpm, 37.0 ± 0.5 °C using 1000ml phosphate buffer pH 6.5.**

| **Time (hr)** | **% of drug dissolved** |
|---|---|
| 1 | 3.9 |
| 2 | 12.0 |
| 4 | 31.6 |
| 6 | 51.1 |
| 8 | 69.5 |
| 10 | 81.6 |
| 12 | 88.5 |
| 16 | 91.1 |
| 20 | 92.2 |
| 24 | 93.3 |

### EXAMPLES 5 TO 8

| | **mg/tab** | | | |
|---|---|---|---|---|
| **Composition** | **Example 5 (reference)** | **Example 6 (reference)** | **Example 7** | **Example 8 (reference)** |
| Quetiapine Fumarate | 230.26 | 230.26 | 230.26 | 230.26 |
| Lactose Monohydrate | 59.87 | 51.12 | 86.12 | 51.12 |
| Microcrystalline Cellulose (Avicel PH 101) | 59.87 | 51.12 | 86.12 | 51.12 |
| Calcium carbonate | - | 100.00 | - | - |
| Magnesium oxide (Light) | - | - | 30.00 | - |
| Sodium bicarbonate | - | - | - | 100.00 |
| Sodium Citrate | 35.00 | - | - | - |
| PVP K 30 | 20.00 | 20.00 | 20.00 | 20.00 |
| A -Carrageenan (Viscarin GP 109^{#}) | 50.00 | 37.50 | 37.50 | 37.50 |
| Dichloromethane ‡ | 166.00 | 166.00 | 166.00 | 166.0 |
| Crospovidone | - | 10.00 | 10.00 | 10.00 |
| Microcrystalline Cellulose (Avicel PH 102) | - | 50.00 | 50.00 | 50.00 |
| Magnesium stearate | 10.00 | 10.00 | 10.00 | 10.00 |
| **Total** | **465.00** | **560.00** | **560.00** | **560.00** |

| | | | | |
|---|---|---|---|---|
| ^{#} Viscarin GP 109 is marketed by FMC. ‡ Evaporates during manufacturing. | | | | |

### Manufacturing process of Example 5

1) Quetiapine fumarate, lactose monohydrate, microcrystalline cellulose (Avicel PH 101), λ -carrageenan, (Viscarin GP 109) and sodium citrate were sifted using from ASTM 40# sieve and mixed using Rapid mixer granulator.
2) The blend of step 1 was granulated using solution of PVP K 30 (of binder addition step) in dichloromethane.
3) The granules of step 2 were dried in Fluidized Bed Dryer and sized using ASTM 20# sieve.
4) Granules of step 3 were lubricated with Magnesium Stearate in conta blender and compressed into a tablet.

### Manufacturing process of Examples 6 and 7

1) Quetiapine fumarate, lactose monohydrate, microcrystalline cellulose (Avicel PH 101), λ -carrageenan (Viscarin GP 109) and calcium carbonate / magnesium oxide (light) were sifted using from ASTM 40# sieve and mixed using Rapid mixer granulator.
2) The blend of step 1 was granulated using solution of PVP K 30 (of binder addition step) in dichloromethane.
3) The granules of step 2 were dried in Fluidized Bed Dryer and sized using ASTM 20# sieve.
4) Granules of step 3 were mixed with crospovidone and microcrystalline cellulose (Avicel PH 102) and lubricated with Magnesium Stearate in conta blender and compressed into a tablet.

### Manufacturing process of Example 8.

1) Quetiapine fumarate, lactose monohydrate, microcrystalline cellulose (Avicel PH 101), λ -carrageenan (Viscarin GP 109) were sifted using from ASTM 40# sieve and mixed using Rapid mixer granulator.
2) The blend of step 1 was granulated using solution of PVP K 30 (of binder addition step) and sodium bicarbonate in dichloromethane.
3) The granules of step 2 were dried in Fluidized Bed Dryer and sized using ASTM 20# sieve.
4) Granules of step 3 were mixed with crospovidone and microcrystalline (Avicel PH 102) cellulose and lubricated with Magnesium Stearate in conta blender and compressed into a tablet.

**Table 5: Dissolution profite* of Example 5 in USP Type I Apparatus (Basket) at 200 rpm, 37.0 ± 0.5 °C**

| **Time (hr)** | **% of drug dissolved** |
|---|---|
| 1 | 9.3 |
| 2 | 19.8 |
| 4 | 41.6 |
| 5 | 53.4 |
| 6 | 60.1 |
| 12 | 91.1 |
| 20 | 95.7 |

| | |
|---|---|
| [*Dissolution Medium: 0- 5 hours: 900 ml of medium containing 0.05 M sodium citrate + 0.09 M sodium hydroxide to provide pH 4.7-4.9; 5-20 hours: addition of 100 ml of medium containing 0.05 M sodium phosphate + 0.46 N sodium hydroxide to provide the pH 6.4-6.8] | |

### EXAMPLES 9

| **Composition** | **mg/tab** |
|---|---|
| **Core** | |
| Quetiapine Fumarate | 57.57 |
| Lactose Monohydrate | 34.30 |
| Microcrystalline Cellulose (Avicel PH 101) | 34.30 |
| *Magnesium oxide* (*light*) | 19.88 |
| Magnesium oxide (light Compacted) | 59.62 |
| PVP K 30 | 17.66 |
| A -Carrageenan (Viscarin GP 209) | 132.50 |
| Dichloromethane ‡ | 120.00 |
| PVP K 30 | 35.33 |
| Magnesium stearate | 8.83 |

| **Coating** | |
|---|---|
| A -Carrageenan (Viscarin GP 209) | 27.50 |
| PEG 400 | 5.77 |
| Titanium dioxide | 5.77 |
| Iron oxide Yellow. | 0.48 |
| Iron oxide Red | 0.48 |
| P. Water‡ | 300.00 |
| Iso propyl alcohol‡ | 232.00 |
| **Total** | **440.00** |

### Manufacturing Process

1. Quetiapine fumarate, Lactose monohydrate, microcrystalline cellulose (Avicel PH 101) magnesium oxide light (compacted), magnesium oxide light and λ - Carrageenan (Viscarin GP 209) were sifted through 40# sieve and mixed using Rapid mixer granulator.
2. Blend of step 1 was granulated using binder solution i.e. PVP K 30 (Intragranular) in dichloromethane.
3. Granules of step 2 were dried using fluid bed dryer.
4. Dried granules of step 3 were sized through 0.5 mm screen of Oscillating granulator.
5. Sized granules of step 4 were mixed with PVP K 30 (extra granular) using blender for 10 minutes.
6. Blend of step 5 was lubricated magnesium stearate using blender for 5 minutes.
7. The lubricated blend of step 6 was compressed to Tablets.
8. λ -Carrageenan (Viscarin GP 209) was dispersed in Isopropyl alcohol followed by Polyethylene Glycol 400.
9. Titanium Dioxide and Ferric oxide yellow were dispersed in Purified Water and passed through colloid mill for 10 minutes.
10. Contents of step 8 to step 9 were mixed.
11. Compressed tablets were coated using A -Carrageenan coating dispersion of step 10.

**Table 6: Dissolution profile of Example 4 in USP Type II Apparatus (Paddle) at 100 rpm, 37.0 ± 0.5 °C using 1000ml phosphate buffer pH 6.5.**

| **Time (hr)** | **% of drug dissolved** |
|---|---|
| 1 | 1.9 |
| 2 | 10.3 |
| 4 | 31.8 |
| 6 | 50.0 |
| 8 | 65.0 |
| 10 | 77.8 |
| 12 | 87.4 |
| 16 | 95.1 |
| 20 | 95.6 |

## Claims

1. A sustained release pharmaceutical composition comprises a core comprising:
- quetispine
- λ-carrageenan as carrageenan,
- Magnesium Oxide as at least one basic agent,
- at least one pharmaceutically acceptable excipient,
and optionally a coating applied on to the core,

2. Sustained release pharmaceutical composition according to claim 1 wherein the core further comprises:
- at least one hydrophobic release-modifying polymer.

3. The compositions according to claims 1 or 2, wherein the coating comprises a polymer selected from a film-forming polymer or a release-modifying polymer.

4. The compositions according to claims 1 or 2, wherein the composition is selected from a tablet, a capsule, a sachet, a granule or a pellet.

5. The compositions according to claims 4, wherein the composition is tablet.

6. The compositions according to claim1, wherein λ -carrageenan is present in an amount from 1 to about 70% of total weight d the composition.

7. The compositions according to claim 1, wherein λ -carrageenan is present in an amount from 1 to about 50% of total weight of the composition.

8. The compositions according to claim 1, wherein λ -carrageenan is present in an amount from 1 to about 30% of total weight of the composition

9. A process for preparing the composition of claim 1 or 2, wherein the process comprises the steps of:
(i) mixing quetlapine carrageenen and at least one pharmaceutically acceptable excipient:
(ii) granulating the mixture of step (i) with a solvent;
(iii) drying the granules;
(iv) optionally coating the granules obtained in step (iii);
(v) mixing the granules of step (iii) or step (iv) with at least one pharmaceutically acceptable excipient, and
(vi) compressing the mixture of step (v) into a tablet.
(vii) optionally coating the table obtained in step (vi)

10. A process for preparing the composition of claim 1 or 2, wherein the precess comprises the steps of:
(i) mixing quetlapine, carrageenan and at least one pharmaceutically acceptable excipient;
(ii) optimally dry- granulating the mixture of step
(iii) optionally coating the granules obtained In step (ii);
(iv) mixing the mixture of step (I) or the granulas of step (ii) or step (iii) with at least one pharmaceutically acceptable excipient, and
(v) compressing the mixture of step (iv) into a tablet.
(vi) optionally coating the tablet obtained in step (v),

## Patentansprüche

1. Pharmazeutische Zusammensetzung mit anhaltender Freisetzung, die einen Kern umfasst, der enthält:
- Quetiapin,
- γ-Carrageen als Carrageen,
- Magnesiumoxid als mindestens einen basische Grundstoff,
- mindestens ein pharmazeutisch akzeptabler Hilfsstoff, und wahlweise eine auf dem Kern aufgebrachte Beschichtung.

2. Pharmazeutische Zusammensetzung mit anhaltender Freisetzung nach Anspruch 1, wobei der Kern ferner umfasst:
- mindestens ein hydrophobes Freisetzung-modifizierendes Polymer.

3. Zusammensetzung nach Anspruch 1 oder 2, wobei die Beschichtung ein filmbildendes Polymer oder ein Freisetzung-modifizierendes Polymer umfasst.

4. Zusammensetzung nach Anspruch 1 oder 2, wobei die Zusammensetzung eine Tablette, eine Kapsel, ein Päckchen, ein Granulat oder ein Pellet ist.

5. Zusammensetzung nach Anspruch 4, wobei die Zusammensetzung eine Tablette ist.

6. Zusammensetzung nach Anspruch 1, wobei γ-Carrageen in einer Menge von 1 bis etwa 70% des Gesamtgewichts der Zusammensetzung vorhanden ist.

7. Zusammensetzung nach Anspruch 1, wobei γ-Carrageen in einer Menge von 1 bis etwa 50% des Gesamtgewichts der Zusammensetzung vorhanden ist.

8. Zusammensetzung nach Anspruch 1, wobei γ-Carrageen in einer Menge von 1 bis etwa 30% des Gesamtgewichts der Zusammensetzung vorhanden ist.

9. Verfahren zur Zubereitung der Zusammensetzung nach Anspruch 1 oder 2, wobei das Verfahren die Schritte umfasst:
(i) Mischen von Quetiapin, Carrageen und mindestens eines pharmazeutisch akzeptablen Hilfsstoffs;
(ii) Granulieren der Mischung aus Schritt (i) mit einem Lösungsmittel;
(iii) Trocknen des Granulats;
(iv) wahlweise Beschichten des in Schritt (iii) erhaltenen Granulats;
(v) Mischen des Granulats aus Schritt (iii) oder Schritt (iv) mit mindestens einem pharmazeutisch akzeptablen Hilfsstoff; und
(vi) Pressen der Mischung aus Schritt (v) in eine Tablette;
(vii) wahlweise Beschichten der in Schritt (vi) erhaltenen Tablette.

10. Verfahren zur Zubereitung der Zusammensetzung nach Anspruch 1 oder 2, wobei das Verfahren die Schritte umfasst:
(i) Mischen von Quetiapin, Carrageen und mindestens eines pharmazeutisch akzeptablen Hilfsstoffs;
(ii) idearleweise Trockengranulieren der Mischung aus Schritt (i);
(iii) wahlweise Beschichten des in Schritt (ii) erhaltenen Granulats;
(iv) Mischen der Mischung aus Schritt (i) oder des Granulats aus Schritt (ii) oder Schritt (iii) mit mindestens einem pharmazeutisch akzeptablen Hilfsstoff; und
(v) Pressen der Mischung aus Schritt (iv) in eine Tablette,
(vi) wahlweise Beschichten der in Schritt (v) erhaltenen Tablette.

## Revendications

1. Composition pharmaceutique à libération prolongée comprenant un noyau comprenant :
- de la quétiapine,
- du γ-carraghénane en tant que carraghénane,
- de l'oxyde de magnésium en tant qu'au moins un agent basique
- au moins un excipient pharmaceutiquement acceptable,
et éventuellement un enrobage appliqué sur le noyau.

2. Composition pharmaceutique à libération prolongée selon la revendication 1 dans laquelle le noyau comprend en outre :
- au moins un polymère hydrophobe modifiant la libération.

3. Composition selon les revendications 1 ou 2, dans laquelle l'enrobage comprend un polymère choisi parmi un polymère filmogène ou un polymère modifiant la libération.

4. Composition selon les revendications 1 ou 2, la composition étant choisie parmi un comprimé, une gélule, un sachet, une granule ou une pastille.

5. Composition selon la revendication 4, ladite composition étant un comprimé.

6. Composition selon la revendication 1, dans laquelle le λ-carraghénane est présent en une quantité allant de 1 à environ 70 % du poids total de la composition.

7. Composition selon la revendication 1, dans laquelle le λ-carraghénane est présent en une quantité allant de 1 à environ 50 % du poids total de la composition.

8. Compositions selon la revendication 1, dans lesquelles le λ-carraghénane est présent en une quantité allant de 1 à environ 30 % du poids total de la composition.

9. Procédé de préparation de la composition de la revendication 1 ou 2, ledit procédé comprenant les étapes de :
(i) mélange de la quétiapine, du carraghénane et d'au moins un excipient pharmaceutiquement acceptable ;
(ii) granulation du mélange de l'étape (i) avec un solvant ;
(iii) séchage des granules ;
(iv) éventuellement enrobage des granulés obtenus à l'étape (iii) ;
(v) mélange des granules de l'étape (iii) ou de l'étape (iv) avec au moins un excipient pharmaceutiquement acceptable ; et
(vi) compression du mélange de l'étape (v) en un comprimé
(vii) éventuellement enrobage du comprimé obtenu à l'étape (vi)

10. Procédé de préparation de la composition de la revendication 1 ou 2, ledit procédé comprenant les étapes de :
(i) mélange de la quétiapine, du carraghénane et d'au moins un excipient pharmaceutiquement acceptable ;
(ii) granulation sèche du mélange de l'étape (i) d'une façon optimale
(iii) éventuellement enrobage des granulés obtenus à l'étape (ii) ;
(iv) mélange du mélange de l'étape (i) ou des granulés de l'étape (ii) ou de l'étape (iii) avec au moins un excipient pharmaceutiquement acceptable ; et
(v) compression du mélange de l'étape (iv) en un comprimé,
(vi) éventuellement enrobage du comprimé obtenu à l'étape (v).
